# EUROPÄISCHE PATENTSCHRIFT

(11) **EP 0 534 265 B1**
(45) Veröffentlichungstag und Bekanntmachung des Hinweises auf die Patenterteilung: **05.04.1995**
(21) Anmeldenummer: 92115697.2
(22) Anmeldetag: 14.09.1992
(51) Int. Cl.: A01N 63/04, C12N 1/14

(54) **Schneckenbekämpfungsmittel**
Molluscicides
Molluscicides

(30) Priorität: 26.09.1991 DE 4132082
(43) Veröffentlichungstag der Anmeldung: 31.03.1993
(73) Patentinhaber: BAYER AG, 51368 Leverkusen (DE)
(72) Erfinder: Stenzel, Klaus, Dr., W-4000 Düsseldorf 13 (DE); Schnorbach, Hans-Jürgen, Dr., W-4019 Monheim (DE); Andersch, Wolfram, Dr., W-5000 Köln 60 (DE)

(56) Entgegenhaltungen:
- EP-A- 0 268 177
- WO-A-91/00012
- WO-A-91/01642
- FR-A- 1 133 721
- CHEMICAL PATENTS INDEX, DOCUMENTATION ABSTRACTS JOURNAL Section Ch, Week 9128, 11. September 1991 Derwent Publications Ltd., London, GB; Class C, AN 91-204731/28
- CENTRAL PATENTS INDEX, BASIC ABSTRACTS JOURNAL Section Ch, Week D44, 23. Dezember 1981 Derwent Publications Ltd., London, GB; Class C, AN 80453 D/44
- Gesunde Pflanzen 33 (2), 30-36 (1981)

## Beschreibung

Die vorliegende Erfindung betrifft die neue Verwendung von pilzlichen Mikroorganismen zur Bekämpfung von Schnecken, Schädlingsbekämpfungsmittel, welche solche Mikroorganismen enthalten sowie neue Mikroorganismen-Stämme.

Schnecken stellen als Schädlinge in Gartenbau und Landwirtschaft ein zunehmendes Problem dar. Sie können durch Fraß schwerwiegende Pflanzenschäden verursachen und auch unerwünschte Verunreinigungen durch Schneckenschleim und Kot herbeiführen. Zur Bekämpfung von Schnecken stehen derzeit nur Mittel zur Verfügung, die chemische Wirkstoffe enthalten. Diese Mittel sind in ihrer Wirksamkeit nicht immer voll befriedigend und können aufgrund der in ihnen enthaltenen Wirkstoffe in ihrer Anwendbarkeit eingeschränkt sein. Es besteht somit ein starkes Bedürfnis nach neuen biologischen Schneckenbekämpfungsmitteln, welche eine gute Wirksamkeit aufweisen und für Anwender und Umwelt besonders einfach und unproblematisch einsetzbar sind.

Es wurde nun gefunden, daß zur Bekämpfung von Schnecken (Mollusken) pilzliche Mikroorganismen, welche eine molluskizide Wirksamkeit aufweisen, verwendet werden können. Diese Mikroorganismen können als solche oder aber in der Form von formulierten Mitteln als molluskizide Schädlingsbekämpfungsmittel (Schneckenbekämpfungsmittel) eingesetzt werden.

Dieser Befund ist überraschend, da im Stand der Technik ausdrücklich darauf hingewiesen wird, daß Krankheiten und ihre Erreger bei Mollusken wohl kaum Bedeutung für eine intensive biologische Bekämpfung erlangen werden (vergleiche D. Godan: Schadschnecken und ihre Bekämpfung, Stuttgart, Ulmer-Verlag 1979, sowie Gesunde Pflanzen 33 (2), 30-36 (1981). In der WO-A- 91 01642 wird ein Verticillium chlamydosporium-Stamm zur Bekämpfung von Nematoden beschrieben, wobei sich kein Hinweis auf Schnecken findet. Die EP-A- 268 177 betrifft trägerfreie Zellgranulate aus Mikroorganismen, u. a. von Verticillium- und Gliocladium-Arten zur Schädlingsbekämpfung, ohne daß auf die Möglichkeit der Schneckenbekämpfung hingewiesen wird. Die WO-A- 91 00012 befaßt sich mit der Schädlingsbekämpfung mit Hilfe von Cyanid-produzierenden Mikroorganismen, insbesondere Bakterien.

Für die erfindungsgemäße Verwendung kommen alle pilzlichen Mikroorganismen in Frage, die eine molluskizide Wirkung aufweisen, das heißt, die bei Kontakt und/oder oraler Aufnahme durch die Schnecken diese in ihrem Fraßverhalten, ihrer Bewegungsfähigkeit, ihrer Fortpflanzung und/oder in ihrer Lebensfähigkeit negativ beeinflussen, wobei eine Abtötung der Schnecken besonders erwünscht ist. Geeignete pilzliche Mikroorganismen mit molluskizider Wirksamkeit können durch den Fachmann anhand von einfachen Routineversuchen (vergleiche z.B. die weiter unten aufgeführten Versuchsbeschreibungen) aufgefunden und isoliert werden.

Bevorzugte erfindungsgemäß verwendbare Pilze sind molluskizid wirksame Pilze aus der Ordnung Hyphomycetales, besonders bevorzugt der Familie Moniliaceae und ganz besonders bevorzugt der Gattungen Gliocladium und Verticillium. Aus diesen Gattungen werden die Arten Gliocladium nigrovirens und Gliocladium solani sowie Verticillium chlamydosporium bevorzugt, wobei als besonders bevorzugte Stämme Gliocladium nigrovirens F 0464 und Gliocladium solani F 0463 aufgeführt werden. Diese Stämme sind neu und Teil der vorliegenden Erfindung. Zur Erfindung gehören auch die Mutanten der genannten Stämme, soweit sie noch die erfindungswesentlichen Eigenschaften (also molluskizide Eigenschaften) aufweisen.

Auch der bereits bekannte Stamm Verticillium chlamydosporium F 0323 weist sehr gute molluskizide Eigenschaften auf. Dieser Stamm ist aus der Sammlung der Collection of the Commonwealth Mycological Institute erhältlich.

Die erfindungsgemäßen neuen Stämme wachsen auf einem künstlichen Nährmedium in Form von septierten verzweigten Hyphensträngen. Die Stämme Gliocladium nigrovirens F 0464 und Gliocladium solani F 0463 bilden an Konidienträgern zunächst Verticillium-ähnliche primäre Konidienträger und anschließend penicilliate sekundäre Konidienträger. Gliocladium nigrovirens F 0464 bildet Konidien mit einer dunkelgrünen Pigmentierung und Gliocladium solani F 0463 bildet Konidien mit einer bräunlich cremefarbenen Pigmentierung, wobei die Konidien eine Größe von 4 - 7 »m in der Länge und 3 -4 »m in der Breite aufweisen. Der Stamm Verticillium chlamydosporium F 0323 bildet am weißlichen Myzel an Trägern Konidien in schleimigen Tröpfchen. Die Größe der Konidien beträgt 3 - 4 »m in der Länge und 1,5 - 2 »m in der Breite. Im Myzel werden typischerweise Dictyochlamydosporen gebildet, die sich aus mehreren dickwandigen Zellen zusammensetzen und deren Durchmesser insgesamt 20 - 25 »m beträgt. Die neuen Stämme sowie der Stamm Verticillium chlamydosporium F0 323 wurden beim Centraalbureau voor Schimmelcultures (CBS), Oosterstraat 1, NL-3740 AG Baarn, Niederlande in Übereinstimmung mit den Bestimmungen des Budapester Vertrages über die internationale Anerkennung der Hinterlegung von Mikroorganismen für die Zwecke von Patentverfahren hinterlegt:

| Stamm | Hinterlegungsdatum | Hinterlegungsbezeichnung |
|---|---|---|
| Gliocladium solani F 0463 | 16. August 1991 | CBS 458.91 |
| Gliocladium nigrovirens F 0464 | 16. August 1991 | CBS 457.91 |
| Verticillium chlanydosporium F 0323 | 16. August 1991 | CBS 459.91 |

Die erfindungsgemäß zu verwendenden Mikroorganismen werden in üblicher Weise durch Fermentation in geeigneten Nährmedien und anschließende Isolierung gewonnen. Hierbei werden die allgemein üblichen Verfahren und Methoden der Mikrobiologie sowie der Biotechnologie eingesetzt.

Die Fermentation soll durch die folgenden beispielhaften Ausführungen erläutert werden.

Die Fermentation kann mit Hilfe fester, halbfester oder flüssiger Nährmedien durchgeführt werden. Vorzugsweise werden wäßrig-flüssige Nährmedien verwendet.

Die Beimpfung der Nährmedien erfolgt nach allgemein üblichen Methoden, z.B. über Platten-, Schrägröhrchen- oder Kolbenkulturen.

Die Kultur erfolgt unter aeroben Bedingungen und kann gemäß den allgemein üblichen Methoden wie unter Verwendung von Oberflächenkulturen, Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Kulturen kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen, insbesondere von Pilzen verwendet werden. Das Nährmedium muß eine oder mehrere assimilierbare Kohlenstoffquellen und Stickstoffquellen sowie Mineralsalze enthalten, wobei diese Produkte in Form von komplexen Gemischen, wie sie insbesondere biologische Produkte verschiedenen Ursprungs darstellen, vorliegen können. Als Kohlenstoffquellen kommen alle üblichen Kohlenstoffquellen in Frage. Beispielsweise seien Kohlenhydrate, insbesondere Polysaccharide, wie Stärke oder Dextrine, Disaccharide, wie Maltose oder Rohrzucker, Monosaccharide, wie Glycose oder Xylose, Zuckeralkohole, wie Mannit oder Glycerin sowie natürlich vorkommende Gemische wie Malzextrakt, Melasse oder Molkepulver genannt. Als Stickstoffquellen kommen alle üblichen organischen und anorganischen Stickstoffquellen in Frage. Beispielsweise seien Eiweißstoffe, Eiweißhydrolysate, Aminosäuren, wie Glutaminsäure, Asparaginsäure, Arginin, Lysin, Ornithin oder Serin, Nucleosidbasen, wie Cytosin oder Uracil sowie Sojabohnenmehl, Baumwollsamenmehl, Linsenmehl, Erbsenmehl, lösliche und unlösliche, pflanzliche Proteine, Maisquellwasser, Hefeextrakt, Peptone und Fleischextrakt sowie Ammoniumsalze und Nitrate, z.B. NH₄Cl, (NH₄)₂SO₄, Na NO₃ und KNO₃ aufgeführt. Die Mineralsalze, welche im Nährmedium enthalten sein sollten, liefern z.B. folgende Ionen:
Mg⁺⁺, Na⁺, K⁺, Ca⁺⁺, NH₄⁺, Cl⁻, SO₄⁻⁻, PO₄⁻⁻⁻ und NO₃⁻
sowie Ionen der üblichen Spurenelemente, wie Cu, Fe, Mn, Mo, Zn, Co, Ni. Falls die Kohlenstoff- oder Stickstoffquellen beziehungsweise das verwendete Wasser nicht ausreichend diese Salze beziehungsweise Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Als besonders geeignet für Oberflächenkulturen kann auch ein Biomalz-Medium verwendet werden, welches beispielsweise aus 2-5 Gew.-% Biomalz und 2 Gew.-% Agar-Agar besteht. Besonders bevorzugt wird die Kultivierung auf Platten.

Der pH-Wert der wachsenden Kulturen sollte vorzugsweise zwischen etwa 6 und etwa 8, insbesondere zwischen 6,5 und 7,5 gehalten werden. Ein zu starker pH-Abfall in den sauren Bereichen kann durch Zusätze einer organischen oder anorganischen Base, vorzugsweise von CaCO₃ vermieden werden, bei der sterile organische oder anorganische Säure, z.B. H₂SO₄, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 15°C und etwa 40°C, vorzugsweise zwischen 18°C und 25°C liegen. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgestellt werden.

Das Fermentationsende wird durch die Produktion der Biomasse determiniert. Die Kultivierung wird aus Gründen der Erhaltung der Lebensfähigkeit der Zellen günstigerweise vor oder zum Zeitpunkt der maximalen Biomasseproduktion abgebrochen. Die Festlegung des Zeitpunktes für das Produktionsende kann durch die üblichen Methoden der Biomassebestimmung durch den Fachmann leicht erfolgen. Durch in einfacher Weise ermittelbare fermentationsspezifische Kenndaten, wie pH-Wert, Sauerstoffpartialdruck, Kohlendioxidpartialdruck oder Konzentration an assimilierbaren Nährstoffbestandteilen kann das technische Produktionsverfahren gesteuert werden.

Die Abtrennung der Mikroorganismen von dem Nährmedium kann in üblicher Weise erfolgen, z.B. durch Filtration über einem Sieb oder siebartigen Geweben mit entsprechender Porenweite, durch Filtration, Zentrifugation oder Separation. Zur Vermeidung von Kontaminationen der Biomasse durch unerwünschte Mikroorganismen, die durch ihre Stoffwechselaktivitäten eine Verminderung der Qualität beziehungsweise eine Zerstörung des Produktes verursachen könnten, wird die Konzentrierung der Mikroorganismen (und gegebenenfalls auch die weitere Verarbeitung) zweckmäßigerweise unter sterilen Bedingungen, wie z.B. in sterilisierten Separatoren durchgeführt.

Zur leichteren weiteren Verarbeitung der Mikroorganismen kann es zweckmäßig sein, der zur konzentrierenden Biomasse überlicherweise verwendete Materialien zuzusetzen, die ein Verklumpen des Myzels während des Konzentrierungsverfahrens verhindern. Hierzu eignen sich oberflächenneutralisierende Materialien, wie z.B. die tonartigen Materialien Bentonite, Talkum, Pyrophylite, Celit, Kalk, Kaolin, Attapulgit oder andere synthetische Silikate.

Die Trocknung der Mikroorganismen-Masse erfolgt durch Dehydratation. Hierbei können die üblichen Methoden zur Trocknung von Biomasse mittels Wärmeübertragung durch Konvektion, wie z.B. die Verfahren der Zerstäubungs-, Strom- und Fließbetttrocknung oder mittels Wärmeübertragung durch Kontakt, wie z.B. die Verfahren der Teller-, Schaufel-, Taumel-, Band-, Walzen-, Vakuumkammer- und Vakuumgefriertrocknung eingesetzt werden. Der Trokknungsprozeß kann auch aus einer Kombination von zwei oder mehreren dieser Verfahren bestehen. Die Dehydratation der Mikroorganismen-Masse wird im diskontinuierlichen oder kontinuierichen Verfahren, vorzugsweise jedoch im diskontinuierlichen Verfahren durchgeführt. Die Verfahren sind im einzelnen derart auszugestalten, daß die Lebensfähigkeit der Zellen über einen möglichst langen Zeitraum hinweg gewährleistet ist.

Die erhaltene Mikroorganismen-Masse kann für die Anwendung weiterverarbeitet werden. Sie kann beispielsweise zu Pulvern oder pulverähnlichen Produkten, z.B. durch vorsichtiges Vermahlen, zerkleinert werden. Die gegebenenfalls zerkleinerte Mikroorganismen-Masse kann auch durch Vermischen mit den üblichen Träger- und Hilfsstoffen in formulierte Produkte übergeführt werden. Als Träger- und Hilfsstoffe kommen hierbei alle bei Pflanzenschutzmitteln, insbesondere bei Schneckenbekämpfungsmittel üblicherweise verwendeten Stoffe in Frage, die für die Mikroorganismen nicht toxisch wirken.

Als Trägerstoffe werden geeignete anorganische und/oder organische Materialien eingesetzt, wie körnige Glas- und Gesteinsschäume, z.B. Perlit-Granulate oder native Produkte, z.B. Kleie, Maiskolbenschrot, Sägespäne und Korkschrot. Als Trägerstoffe können bevorzugt auch Fraßstoffe, also die üblicherweise in Schneckenköderformulierungen enthaltenen Lockstoffe und/oder Futterstoffe (also für Schnecken physiologisch verwertbare Substanzen) verwendet werden. Auch Mischungen von Fraßstoffen mit geeigneten anderen organischen und/oder anorganischen Trägerstoffen sind verwendbar.

Als Fraßstoffe kommen vorzugsweise in Betracht: Gemahlenes Getreide, wie z.B. Weizenmehl, Gerstenmehl, Roggenmehl sowie Reisstärke, Sojaschrot, Fischmehl, Melasse, Rapsschrot u.a.. Es kann entweder nur ein Fraßstoff oder auch ein Gemisch von Fraßstoffen vorhanden sein.

Als Hilfsstoffe können beispielsweise Bindemittel, wie Methylcellulose, Polyvinylpyrrolidon, Polyvinylalkohol, Polyacrylate, Polymethacrylate, natürliche Wachse, chemisch veränderte Wachse und synthetische Wachse, Zucker, Dextrin, Stärke, Alginate, Agar-Agar, Ligninsulfonate und Gummi arabicum, Mittel, die die Austrocknung der Präparate verhindern, wie Polyalkohole, z.B. Zucker oder Glycerin, Konservierungsstoffe, Farbstoffe, Schneckenlockstoffe, Warmbluter-Repellents und/oder sonstige Formulierungshilfsstoffe eingesetzt werden. Auch Kombinationen mit bekannten molluskiziden Wirkstoffen, z.B. Metaldehyd oder Mercaptodimethur sind möglich.

Die Schneckenbekämpfungsmittel, welche bevorzugt neben den molluskiziden Mikroorganismen weitere Träger- und/oder Hilfsstoffe enthalten, liegen in ihrer anwendungsfertigen Form vorzugsweise als spritz- oder streubare Pulver, als Granulate (wobei die Mikroorganismen auf die Trägeroberfläche aufgebracht vorliegen oder mit dem Trägermaterial vermischt vorliegen), oder als Pellets vor. Besonders bevorzugte Formulierungen sind streufähige Pulver, Granulate oder Pellets.

Die molluskiziden Pilze können erfindungsgemäß als solche (also ohne Beimischungen) oder in Form von formulierten Produkten (Beimischung von Träger- und Hilfsstoffen) verwendet werden. Vorzugsweise werden formulierte Produkte eingesetzt.

Die formulierten Produkte enthalten vorzugsweise 5 bis 90, insbesondere 20 bis 70 und ganz besonders bevorzugt 30 bis 50 Gewichtsprozente Pilzmasse.

Die Mikroorganismen können jedoch auch in Form eines trägerfreien Granulats, welches gemäß EP-A 0 268 177 erhalten wird, eingesetzt werden.

Die molluskiziden pilzlichen Mikroorganismen können in Form der verschiedenen Lebens- beziehungsweise Entwicklungsstadien eingesetzt werden. So können z.B. das gegebenenfalls fragmentierte Myzel als auch die Sporen (Chlamydosporen, Blastosporen oder Konidien) verwendet werden. Die jeweils optimale Anwendungsform kann der Fachmann anhand einfacher Routineuntersuchungen leicht ermitteln.

Die jeweils als Schneckenbekämpfungsmittel auszubringende Menge ist wegen der fehlenden Warmblütertoxizität unkritisch und richtet sich nach den jeweiligen Gegebenheiten, wie Befallsgrad, Klimabedingungen und zu schützende Pflanzen. Zweckmäßigerweise werden die Schneckenbekämpfungsmittel möglichst gleichmäßig zwischen den Kulturpflanzen durch Aufsprühen einer wäßrigen Suspension oder durch Streuen der Pulver, Granulate oder Pellets auf dem Boden verteilt. Bei nicht dichtem Pflanzenbewuchs kann es auch zweckmäßig sein, um die schützenden Pflanzen "Fangstreifen" anzulegen.

Die erfindungsgemäßen Mittel können zur Bekämpfung von praktisch allen wichtigen pflanzenschädigenden Schnekken, vorzugsweise Landschnecken, verwendet werden.

Gegebenenfalls kann es zweckmäßig sein, verschiedene Mikroorganismenstämme zu kombinieren, um zu einem optimalen Bekämpfungserfolg zu gelangen. Zu den bedeutenden schädlichen Schnecken, welche erfindungsgemäß bekämpft werden können, gehören bei den Landschnecken z.B. die Nacktschnecken Arion rufus (Große Wegschnecke); Arion hortensis und andere Arionidae, Limax-Arten und die Ackerschnecken u.a. Deroceras reticulatum und D. agreste aus der Familie Limacidae, sowie Arten aus der Familie Milacidae, weiterhin die Gattungen Bradybaena, Cepaea, Cochlodina, Discus, Euomphalia, Galba, Helicigona, Helix, Helicella, Helicodiscus, Lymnaea, Opeas, Vallonia und Zonitoides.

Bei den zu bekämpfenden Wasserschnecken können beispielhaft die Schnecken der Gattungen Pomacea und Ampullarius aufgeführt werden.

Wegen der hervorragenden Pflanzenverträglichkeit der erfindungsgemäßen Schneckenbekämpfungsmittel ergeben sich von der Seite der zu schützenden Pflanzen keinerlei Einschränkungen. So können alle Zier- und Kulturpflanzen in Landwirtschaft, Forsten und Gartenbau (auch in Gewächshäusern) in allen Wachstumsstadien vor Schäden durch Schnecken geschützt werden.

Im vorliegenden Text beziehen sich alle Prozentangaben auf Gewichtsprozente, wenn keine andere Angabe erfolgt.

### Beispiel A

### Allgemeine Methode zum Auffinden von molluskiziden Pilzen

Adulte Schnecken, z.B. große Wegschnecken (Arion rufus) werden zur Eiablage in angefeuchtete Erde gesetzt und bei Raumtemperatur gehalten. Eine Woche nach der Ablage werden die Eier gesammelt und intensiv mit sterilem Wasser gespült. Nach Beseitigung aller Erdpartikel und oberflächlich anhaftender Mikroorganismen werden die Eier auf Filterpapier gelegt, so daß der Wasserfilm auf der Eioberfläche abtrocknet.

Die Eier werden anschließend auf Malzextraktagar (Malzextrakt, Glucose, Agar je 20 Gramm in 1000 ml Wasser gelöst, pH 7,0) ausgelegt und bei einer Temperatur von 20°C inkubiert. Die Eier werden in regelmäßigen Abständen unter dem Binokular auf eine Bildung von pilzlichem Mycel hin untersucht.

Die unterschiedlichen Pilzarten werden mit Hilfe eienr feinen Glasnadel von den Eiern auf Malzextraktagar übertragen und bei einer Temperatur von 20°C inkubiert.

Zur Beseitigung eventuell vorhandener Kontaminationen werden mit Hilfe der feinen Glasnadeln einzelne Sporen der Pilze auf frischem Malzextraktagar übertragen und entsprechend inkubiert.

Die molluskizide Wirksamkeit von derart isolierten Mikroorganismen-Stämmen kann gemäß den unten angegebenen biologischen Beispielen geprüft werden.

Nach der obigen Methode wurden die im vorliegenden Text speziell beschriebenen molluskiziden Gliocladium-Stämme isoliert.

Bereits isoliert vorliegende Mikroorganismenstämme (und gegebenenfalls deren Mischungen) können durch die direkte Behandlung von Schnecken mit diesen Mikroorganismen (z.B. gemäß Beispiel 1) auf ihre molluskizide Wirksamkeit getestet werden. Wirksame Stämme können so auf einfache Weise in Routineversuchen ermittelt werden.

### Beispiel B

### Anzucht der molluskiziden Pilze

a) Gewinnung von diffusem Myzel
   Die jeweiligen Pilzisolate, z.B. Pilze der Gattung Gliocladium, werden für die Vermehrung auf Hafermehlagar (Hafermehl, 30 Gramm und Agar, 20 Gramm pro Liter Wasser) bei einer Temperatur von 25°C angezogen.
   Zur Gewinnung von diffusem Myzel werden die Pilzisolate in Schüttelkulturen auf flüssigen Nährmedium (Glucose, Sojamehl, je 10.0 Gramm in 1000 ml Wasser gelöst, pH 7,0) angezogen. Je 100 ml des Nährmedium werden in 1,0 l Erlenmeyerkolben gefüllt und 20 Minuten bei 121°C sterilisiert. Nach dem Abkühlen wird die Nährlösung mit 10⁶ Konidien pro Milliliter Nährlösung angeimpft und bei einer Temperatur von 25°C für 3-4 Tage auf einem Rundschüttler bei 100 Umdrehungen pro Minuten inkubiert. Das gebildete Myzel wird in üblicher Weise, z.B durch Absieben abgetrennt, gewaschen und getrocknet.
b) Die schneckenpathogenen Pilzisolate können auch in Form von Granulate analog zum Verfahren gemäß EP-A 0 268 177 gewonnen werden.
   Die Mikroorganismen werden in Schüttelkolben unter den Bedingungen der Flüssigkultur angezogen. Als Nährmedium wird z.B. für Verticillium clamydosporium eine Kombination aus Fleischextrakt (1,0 %, w/v) und Dextrin (1,0 %, w/v), pH 8,0 bzw. und für die Isolate der Gattung Gliocladium aus Hefeextrakt (1,0 % w/v) und Saccharose (1,0 %, w/v) pH 8,0 gewählt. Je 100 ml der Nährlösung werden in 1,0 l Erlenmeyerkolben gefüllt und 20 Minuten bei einer Temperatur von 121°C sterilisiert. Nach dem Abkühlen wird die Nährlösung mit Konidien, die mit Wasser von Agarplatten abgeschwemmt wurden, in einer Zelldichte von 10⁶ Konidien pro Milliliter Nährlösung angeimpft.

Die Schüttelkulturen werden bei einer Temperatur von 25°C auf einem Rundschüttler bei 100 Umdrehungen pro Minute inkubiert. Nach 3-4 Tagen Inkubation haben sich die als Mikroorganismen Granulate oderr Pellets bezeichneten "Mycelkügelchen" gebildet.

Die Abtrennung der Pellets von der Kulturbrühe und ihre Konservierung erfolgt in üblicher Weise z.B. durcM Abfiltrieren.

Die biologische Wirksamkeit der erfindungsgemäßen Schneckenbekämpfungsmittel soll anhand der folgenden Beispiele erläutert werden.

### Beispiel 1

### Wirkung von Verticillium chlamydosporium F 0 323 gegen Schnecken

Jeder Versuchsansatz wurde mit 5 Individuen der Großen Wegschnecke (Arion rufus) in je 5 einzelnen Versuchsgefäßen durchgeführt. Die Schnecken erhielten einen mit dem Pilz behandelten Köder an sieben aufeinanderfolgenden Tagen zum Fraß vorgelegt. Nach den ersten drei Tagen erhielten die Schnecken Kartoffelscheiben als Alternativfutter. Am 7. Tag wurden die lebenden und toten Schnecken ausgezählt. Die Kontrollgruppe erhielt den gleichen Köder, jedoch ohne Pilz. Während der Versuchsdauer wurde die Temperatur auf 19°C und die relative Luftfeuchte auf 95 % gehalten.

Die Versuchsergebnisse sind in der folgende Tabelle 1 aufgelistet.

**Tabelle 1**

| Eingesetzter Pilz | % Mortalität am 7. Tag |
|---|---|
| Kontrolle | 0 |
| V. chlamydosporium F 0 323 | 60 |

### Beispiel 2

### Wirkung von Gliocladium solani F 0 463 und Gliocladium nigrovirens F 0 464 gegen Schnecken

### Methode siehe Beispiel 1

Die Versuchsergebnisse sind in der folgenden Tabelle 2 aufgelistet.

**Tabelle 2**

| Eingesetzter Pilz | % Mortalität am 7. Tag |
|---|---|
| Kontrolle | 0 |
| G. solani F 0 463 | 80 |
| G. nigrovirens F 0 464 | 80 |

### Beispiel 3

### Wirkung von Verticillium chlamydosporium F 0 323 (trägerfreies Granulat) gegen Schnecken

Jeder Versuchsansatz wurde mit 5 Individuen der Großen Wegschnecke (Arion rufus) in je 5 einzelnen Versuchsgefäßen durchgeführt. Die Schnecken erhielten einen aus dem trägerfreien Pilzgranulat (gemäß EP-A-0 268 177, Beispiel 3) enthaltenden Köder an sieben aufeinanderfolgenden Tagen zum Fraß vorgelegt. Nach den ersten drei Tagen erhielten die Schnecken Kartoffelscheiben als Alternativfutter. Am 10. Tag wurden die lebenden und toten Schnecken ausgezählt. Die Kontrollgruppe erhielt den gleichen Köder, jedoch ohne das Pilzgranulat. Während der Versuchsdauer wurde die Temperatur auf 19°C und die relative Luftfeuchte auf 95 % gehalten.

Die Versuchsergebnisse sind in der folgenden Tabelle 3 aufgelistet.

**Tabelle 3**

| Eingesetzter Pilz | % Mortalität am 10. Tag |
|---|---|
| Versuch 1: | |
| Kontrolle | 0 |
| V. chlamydosporium F 0 323-Granulat | 75 |

| Versuch 2: | |
|---|---|
| Kontrolle | 0 |
| V. chlamydosporium F 0 323-Granulat | 80 |

## Patentansprüche

1. Verwendung von molluskiziden pilzlichen Mikroorganismen zur Bekämpfung von Schnecken durch Kontakt und/oder orale Aufnahme durch die Schnecken.

2. Verwendung von molluskiziden pilzlichen Mikroorganismen der Ordnung Hyphomycetales gemäß Anspruch 1 zur Bekämpfung von Schnecken.

3. Verwendung von molluskiziden pilzlichen Mikroorganismen der Familie Moniliaceae, vorzugsweise der Gattungen Gliocladium und Verticillium gemäß Anspruch 1 zur Bekämpfung von Schnecken.

4. Verwendung von molluskiziden pilzlichen Mikroorganismen der Arten Gliocladium nigrovirens, Gliocladium solani und Verticillium chlamydosporium gemäß Anspruch 1 zur Bekämpfung von Schnecken.

5. Verwendung der molluskiziden pilzlichen Mikroorganismen-Stämme Gliocladium nigrovirens F 0 464 (entsprechend CBS 457.91), Gliocladium solani F 0 463 (entsprechend CBS 458.91) und Verticillium chlamydosporium F 0 323 (entsprechend CBS 459.91) gemäß Anspruch 1 zur Bekämpfung von Schnecken.

6. Schneckenbekämpfungsmittel, enthaltend wenigstens einen der molluskiziden pilzlichen Mikroorganismus-Stämme Gliocladium nigrovirens F 0 464 (entsprechend CBS 457.91) und Gliocladium solani F 0 463 (entsprechend CBS 458.91).

7. Mikroorganismen-Stämme Gliocladium nigrovirens F 0 464 (entsprechend CBS 457.91), Gliocladium solani F 0 463 (entsprechend CBS 458.91) und ihre Mutanten.

## Claims

1. Use of molluscicidal fungal microorganisms for combating slugs and snails by contact with and/or ingestion by the slugs and snails.

2. Use of molluscicidal fungal microorganisms from the order of the Hyphomycetales according to Claim 1 for combating slugs and snails.

3. Use of molluscicidal fungal microorganisms from the family of the Moniliaceae, preferably the genera Gliocladium and Verticillium, according to Claim 1 for combating slugs and snails.

4. Use of molluscicidal fungal microorganisms of the species Gliocladium nigrovirens, Gliocladium solani and Verticillium chlamydosporium according to Claim 1 for combating slugs and snails.

5. Use of the molluscicidal fungal microorganism strains Gliocladium nigrovirens F 0 464 (corresponding to CBS 457.91), Gliocladium solani F 0 463 (corresponding to CBS 458.91) and Verticillium chlamydosporium F 0 323 (corresponding to CBS 459.91) according to Claim 1 for combating slugs and snails.

6. Molluscicides, containing at least one of the molluscicidal fungal microorganism strains Gliocladium nigrovirens F 0 464 (corresponding to CBS 457.91) and Gliocladium solani F 0 463 (corresponding to CBS 458.91).

7. Microorganism strains Gliocladium nigrovirens F 0 464 (corresponding to CBS 457.91), Gliocladium solani F 0 463 (corresponding to CBS 458.91) and their mutants.

## Revendications

1. Utilisation de micro-organismes molluscicides de la classe des champignons pour combattre des mollusques par contact et/ou par ingestion orale par les mollusques.

2. Utilisation de micro-organismes molluscicides de la classe des champignons, de l'ordre des hyphomycétales suivant la revendication 1, pour combattre des mollusques.

3. Utilisation de micro-organismes molluscicides de la classe des champignons, de la famille des Moniliaceae, de préférence des genres Gliocladium et Verticillium suivant la revendication 1 pour combattre des mollusques.

4. Utilisation de micro-organismes molluscicides de la classe des champignons, appartenant aux espèces Gliocladium nigrovirens, Gliocladium solani et Verticillium chlamydosporium, suivant la revendication 1 pour combattre des mollusques.

5. Utilisation des souches de micro-organismes molluscicides de la classe des champignons, Gliocladium nigrovirens F 0 464 (correspondant à CBS 457.91), Gliocladium solani F 0 463 (correspondant à CBS 458.91) et Verticillium chlamydosporium F 0 323 (correspondant à CBS 459.91), suivant la revendication 1 pour combattre des mollusques.

6. Compositions molluscicides, contenant au moins l'une des souches molluscicides de micro-organismes, de la classe des champignons, Gliocladium nigrovirens F 0 464 (correspondant à CBS 457.91) et Gliocladium solani F 0 463 (correspondant à CBS 458.91).

7. Les souches de micro-organismes Gliocladium nigrovirens F 0 464 (correspondant à CBS 457.91), Gliocladium solani F 0 463 (correspondant à CBS 458.91), et leurs mutants.
